# EUROPEAN PATENT SPECIFICATION

(11) **EP 1 003 482 B1**
(45) Date of publication and mention of the grant of the patent: **27.04.2005**
(21) Application number: 98932506.3
(22) Date of filing: 09.07.1998
(51) Int. Cl.: A61K 9/16

(54) **MICROPARTICLES FOR CONTROLLED DELIVERY OF BIOLOGICALLY ACTIVE MOLECULES**
MIKROPARTIKEL ZUR GESTEUERTEN WIRKSTOFFABGABE BIOLOGISCH AKTIVER MOLEKÜLE
MICROPARTICULES PERMETTANT DE LIBERER DE MANIERE REGULEE DES MOLECULES ACTIVES SUR LE PLAN BIOLOGIQUE

(30) Priority: 10.07.1997 IT RM970418
(43) Date of publication of application: 31.05.2000
(73) Proprietor: ISTITUTO SIEROVACCINOGENO ITALIANO I.S.I. S.p.A., 55020 Castelvecchio Pascoli (Lucca) (IT)
(72) Inventor: COWDALL, Jenny, Shrewsbury Shropshire SY3 0NZ (GB); DAVIES, John, Shrewsbury Shropshire SY3 0NZ (GB); ROBERTS, Mark, Copthorne Shrewsbury Shropshire (GB); CARLSSON, Anders, S-112 38 Stockholm (SE); SOLARO, Roberto, I-56100 Pisa (IT); MAZZANTI, Giuseppe, I-55020 Castel Vecchio Pascoli (IT); CHIELLINI, Elisabetta, I-56100 Pisa (IT); CHIELLINI, Federica, I-56100 Pisa (IT); SÖDERLIND, Erik, S-431 37 Mölndal (SE)
(74) Representative: Gervasi, Gemma, Dr.
(86) International application number: PCT/IT1998/000192
(87) International publication number: WO 1999/002135

(56) References cited:
- EP-A- 0 509 968
- WO-A-91/15193
- WO-A-93/25221
- WO-A-94/23738
- OHYA Y ET AL: "PREPARATION OF ALBUMIN MICROSPHERES GRAFTED GALACTOSE RESIDUES THROUGH POLYETHYLENE-GLYCOL SPACERS, RELEASE BEHAVIOR OF 5-FLUOROURACIL FROM THEM, AND THEIR LECTIN-MEDIATED AGGREGATION" JOURNAL OF MACROMOLECULAR SCIENCE: PART A - CHEMISTRY, vol. A28, no. 8, 1991, pages 743-760, XP002060122

## Description

This invention relates to microparticles for controlled delivery of biologically active molecules and for their employment for *in vitro* and *in vivo* therapy, prophylaxis and diagnostics.

More particularly, this invention relates to microparticles featuring controlled and targeted delivery of biologically active molecules like medicaments, anti-bodies, tracers etc.

Prior art mentions numerous strategies for the targeted conveyance of an *in vitro* and *in vivo* biologically active material in a specific way, S.S. Davis; L. Illum, Targeting of Drugs 4, Ed. by G. Gregoriadis, Plenum Press, N.Y. (1994), pg. 183 and ff., Targeted Therapeutic Systems, Ed. by P. Tyle; B.P. Ram, Marcel Dekker Inc. (1990).

The Authors of this invention have set forth a new procedure for the preparation of particles deriving from the interaction of proteins with polymers, which particles are controlled both in shape and size and are particularly advantageous for targeted conveyance of biologically active compounds.

The specific targeting to liver parenchymal cells has been obtained for instance by employing molecules of different structures and origins, as for example proteins modified with carbohydrate components, M. Monsigny et al., Advanced Drug Delivery Reviews 14: 1 - 24 (1994), or with proteins containing carbohydrates exposed on their surfaces, as for instance asialofetuin and asialoroso-mucoid, H. Ibrihara, Pharmac. Res. 7,: 542 - 546 (1990).

Various glycolipids, like asialogangliosides, ceramides, lactosylceramides, have been employed mainly for the preparation of liposomes or of other hydrophobic microparticle systems, Leserwan Lee; P. Machy, Liposomes from biology to therapeutics, Ed. by Marc J. Ostro, Marcel Dekker.

WO 93/25221 refers to biodegradable polymer microspheres, containing a core material surrounded by a polymer membrane capsule.

Also synthetic polymers having carbohydrate components are well known in the prior art and are employed as agents for coating surfaces, K. Sugiyama; Tokn, Polym. J. 27: 179 - 188 (1995), N. Adachi et al., J. Biomater. Sci. Polymer Ed. 6: 463 - 479 (1994), as means for targeted conveyance in a specific way of biologically active molecules which are linked to said means by covalent bonds, Groman E. et al., PCT Int. App. WO 95/34325.

As a result of their hydrophobic properties, many materials are not suitable for the targeted conveyance of hydrophilic molecules.

This invention relates to microparticles made up of a proteic component and a polymeric component, both of them having an outside superficial layer consisting of molecules which have the property of interacting selectively with other molecules present on the reactive receptive sites. More particularly, the particles of this invention are able to incorporate and convey biological active material toward target cells, tissues or organs and to deliver such material in a time-programmed way. Accordingly, the particles of this invention can be applied to the fields of therapy, prophylaxis and diagnostics, both *in vitro* and *in vivo.*

The microparticles of this invention overcome the disadvantages of prior art which are due to poor compatibility or lack of compatibility among the materials that the particles are made up of and the biologically active material. Indeed, the efficiency of trapping of the biologically active material which is almost always water soluble and hydrophilic is not optimal in the presence of materials of hydrophobic nature.

Accordingly, this invention supplies a high load-loading of hydrophilic biologically active molecules, so as to supply them also with a higher stability, in particular if they are proteins or peptides which are sensitive to heat, solvents, steric and/or environmental interferences.

The biologically active material, preferably of proteic kind, is trapped within a homogeneous net which is formed as an effect of non-covalent and/or ionic-type interactions between an essentially proteic component and an essentially polymeric component, which net is capable of trapping in particular water-soluble materials and of keeping their structural and biological characteristics.

The process for obtaining the microparticles of this invention consists in the controlled co-precipitation of a solution of a synthetic, semisynthetic or natural polyanion and of a protein solution containing the active principle, as already described in the co-pending patent application simultaneously filed by the same Authors, and in the next step of coating with a layer of a component capable of specific and selective interactions with receptive and reactive sites.

Accordingly, it is an object of this invention a chemically homogeneous microparticle for controlled delivery of biologically active molecules which is made up of a core comprising a proteic component and a natural, synthetic or semisynthetic polymer, said proteic component and said polymer having non-covalent and/or ionic-type interactions and forming a homogenous net and an outside layer made up of natural, synthetic or semisynthetic molecules which can be recognized by receptors or components of the cell surface of living organisms, or which are capable of recognizing natural, synthetic or semisynthetic molecular structures; the microparticle having a size between 100 nm and 1.000 nm, preferably between 100 nm and 400 nm, whith a monomodal distribution and limited-value polydispersion index.

Preferably the essentially proteic component is made up of at least a protein selected from albumin (human, bovine, or egg albumin), alpha 1-globulin, alpha 2-globulin, collagen, fibrinogen, gelatine, and more preferably natural or recombinant human albumin.

According to a particular embodiment, the core also comprises modified cyclodextrins.

Preferably the natural, synthetic or semisynthetic polymer consists of polyanionic polymers and their mixtures, more preferably the polyanionic polymers contain carboxyl and/or sulfonic and/or phosphoric groups.

In a particular embodiment of the invention, the polymer consists of hemiesters of alternate copolymers of maleic anhydride with alkylvinyl ether oxyethylenglycols with polverization degree 1≤n≤1000, and preferably 1≤n≤10.

The outside layer is made up preferably of glycolipids and/or glycoproteins and/or synthetic or semisynthetic polymers containing glycoside or saccharide residues, and/or mono- and/or oligosaccharides, more particularly the glycoside residues are galactose, lactose or mannose residues.

According to a particular embodiment, the outside layer is made up of polyamino acids containing galactose, lactose or mannose residues.

Glycolipids are preferably made up of mono- or di-galactosyldiglycerides, gangliosides, lactosylcerebrosides, neogalactolipids (lipids modified by means of saccharide residues).

Glycoproteins are preferably made up of asialo-glycoproteins and neoglycoproteins, more preferably said asialoglycoproteins comprise asialofetuin, asialooroso-mucoid, asialotransferrin. Alternatively, glyco-proteins comprise albumin glycosylate, in particular galactosylate, lactosylate, mannosylate.

According to a particular embodiment, mono- and oligosaccharides are made up of galactose, lactose and/or mannose.

Receptors or components of the cell surface of living organisms are preferably expressed in normal or in pathological conditions on hepatic cells, parenchymal or not parenchymal, or in bone marrow, and more preferably the receptors are of the selectinic E or P type.

The biological active molecules are preferably made up of peptides, polypeptides, proteins, carbohydrates, nucleic acids, lipids or polysaccharides, of natural, synthetic or semisynthetic origin, and more preferably the nucleic acids comprise polynucleotides, antisense oligonucleotides, molecular conjugates containing RNA or DNA, hydrosoluble RNA and DNA vectors. More preferably, the polypeptides comprise cytochins, lymphochins, monochins, interferons, and even more preferably the interferon is natural, synthetic or semi-synthetic alpha-interferon.

According to a particular embodiment, the biologically active molecules are a natural or recombinant vaccine.

According to a particular embodiment, the biologically active molecules are natural or recombinant bacterial or viral antigens.

According to a particular embodiment, the biologically active molecules are an immunological adjuvant.

According to a particular embodiment, the biologically active molecules are made up of or are linked to natural or recombinant antibodies and/or fragments of the same.

According to a particular embodiment, the biologically active molecules are made up of a radioactive agent, an enzyme, a coagulation factor, or a hydrosoluble hormone, and preferably the enzyme is the superoxide-dismutase, even modified, and preferably the coagulation factor is the factor VIII.

According to a particular embodiment, the biologically active molecules are made up of natural, synthetic or semisynthetic compounds belonging to antitumorals, antibiotics, antiinflammatories, immuno-stimulants, immunosuppressive agents.

Preferably the targeting toward specific organs is carried into effect on the basis of the average size of the microparticles.

It is a further object of this invention a process for the preparation of chemically homogeneous microparticles by:
- mixing the organic hydroalcoholic water solution of a natural, synthetic or semisynthetic polymer with a protein solution;
- carrying out a controlled co-precipitation at a pH between 3 and 7, preferably between 4 and 5 at a temperature variable between 0°C and 70°C, preferably between 10°C and 40°C;
- adding the component of the outside layer under stirring conditions:
- stirring until evaporation of the organic solvent.

It is a further object of this invention a pharmaceutical composition comprising the microparticles dispersed within a pharmaceutically acceptable medium. Preferably the composition is in the form of a lyophilized material to be reconstituted, or of a water suspension or of a gel. The composition is intended for administration through injection and/or implantation by the subcutaneous, intradermal, intrathecal, intramuscular, intraperitoneal, intravenous, and the intra-arterial routes.

This invention will be described now by means of examples of the same which are not intended to be limitative of it.

### Example 1

### Preparation of PAM14/HSA microspheres

A solution of 100 mg of the butyl hemiester of the alternate copolymer of maleic anhydride/methoxyethyl vinyl ether (PAM14) in 3.0 ml of a 4:1 mixture of acetone/water was dropped by means of a syringe fitted with a 22G needle into a solution of 33 mg of human seroalbumin (HSA) in 2.5 ml of bidistilled water that was kept under magnetic stirring at room temperature. When the addition was finished, stirring was kept on for the time necessary to evaporate the organic solvent completely. Dimensional analysis by means of dynamic light scattering showed that the microspheres were characterised by an average diameter of 130 nm and by a polydispersion index of 0.1 - 0.3.

### Example 2

### Preparation of PAM14/HSA/HGL microspheres

A solution of 50 mg of the butyl hemiester of the alternate copolymer of maleic anhydride/methoxyethyl vinyl ether (PAM14) into 3.0 ml of a mixture of acetone/water 4/1 was dropped by means of a syringe fitted with a 22G needle into a solution of 36 mg of human seroalbumin (HSA) in 3.0 ml of bidistilled water that was kept under magnetic stirring. A dispersion of 6.7 mg of digalactosyldiacylglycerol hydrogenated (HDGDG) in 3.0 ml of water was added under strong magnetic stirring to the dispersion of microspheres so obtained. When the addition was finished, stirring was kept on for the time needed to evaporate the organic solvent completely. Dimensional analysis by dynamic light scattering showed that the microspheres were characterised by an average diameter of 150 nm and a poly-dispersion index of 0.1 - 0.3.

### Example 3

### Preparation of PAM11/HSA/DGDG microspheres

A solution of 60 mg of the butyl hemiester of the alternate copolymer of maleic anhydride/methoxyethyl vinyl ether (PAM11) in 2.0 ml of water was dropped by means of a syringe fitted with a 22G needle into a solution of 24 mg of human seroalbumin (HSA) in 2.0 ml of bidistilled water kept under magnetic stirring. A dispersion of 5.5 mg of digalactosyldiacylglycerol (DGDG) in 2.0 ml of water was added to the dispersion of the microspheres under magnetic stirring. When the addition was finished, magnetic stirring was kept for a further period of 60 minutes. Dimensional analysis by means of dynamic light scattering showed that the microspheres were characterized by an average diameter of 150 nm and by a polydispersion index of 0.1 - 0.3.

### Example 4

### Preparation of PAM11/MYO/HSA/DGDG microspheres

A solution of 100 mg of the methyl hemiester of the alternate copolymer of maleic anhydride/methoxyethyl vinyl ether (PAM11) in 1,0 ml of a 1/1 mixture of ethanol/water and a solution of 10 mg of digalactosyl-diacylglycerol (DGDG) in 2.0 ml of water were simultaneously dropped by means of two syringes both fitted with a 22G needle into a solution of 50 mg of a mixture of myoglobin/human seroalbumin (MYO/HSA) 1/4 in 8 ml of bidistilled water under magnetic stirring. When the addition was finished, magnetic stirring was kept for a further period of 60 minutes. Dimensional analysis by means of dynamic light scattering showed that the microspheres were characterized by an average diameter of 500 nm and by a polydispersion index of 0.1 - 0.3.

### Example 5

### Preparation of PMA14/HSA, HSA-FITC/DGDG/MN microspheres

A solution of 210 mg of the butyl hemiester of the alternate copolymer of maleic anhydride/methoxyethyl vinyl ether (OAM14) in 4.0 ml of a 4/1 mixture of ethanol/water was dropped by means of a syringe fitted with a 22G needle into a solution of 40 mg of human seroalbumin (HSA) and 600 mg of human seroalbumin fluoresceinisothiocyanate (HSA-FITC) in 10 ml of bi-distilled water containing 10% by weight of mannitol (MN). A dispersion of 28 mg of digalactosyldiacylglycerol (DGDG) in 3.0 ml of water was added to the dispersion of microspheres. When the addition was finished, magnetic stirring was kept for a further period of 90 minutes. Dimensional analysis by means of dynamic light scattering showed that the microspheres were characterized by an average diameter of 150 nm and by a polydispersion index of 0.1 - 0.3.

### Example 6.

### Preparation of PAM14/HSA/HSA-FITC/DGDG/GDA-bCD microspheres

A solution of 200 mg of the butyl hemiester of the alternate copolymer of maleic anhydride/methoxyethyl vinyl ether (PAM14) in 4.0 ml of a 3/2 mixture of ethanol/water was dropped by means of a syringe fitted with a 22G needle into a solution of 40 mg of human seroalbumin (HSA) and 486 mg of human seroalbumin fluoresceinisothiocyanate (HSA-FITC) in 10.0 ml of bidistilled water containing 5% by weight of glycidyl-di-isopropylidenearabitol-betacyclodextrin (GDA-bCD). A dispersion of 21 mg of digalactosyldiacylglycerol (DGDG) in 3.0 ml of bidistilled water was added under magnetic stirring to the dispersion of the microspheres. When the addition was finished, the dispersion was kept under magnetic stirring for a further period of 60 minutes. Dimensional analysis by means for dynamic light scattering showed that the microspheres were characterized by an average diameter of 200 nm and by a polydispersion index of 0.1 - 0.2.

### Example 7

### Preparation of microspheres of PAM14/HSA, HSA-FITC/DGDG/GDX-bCD)

A solution of 100 mg of the butyl hemiester of the alternate copolymer of maleic anhydride/methoxyethyl vinyl ether (PAM14) in 2.0 ml of a 3/2 mixture of ethanol/water was dropped by means of a syringe fitted with a 22G needle into a solution of 20 mg of human seroalbumin (HSA) and 243 mg of human seroalbumin fluoresceinisothiocyanate (HSA-FITC) in 5.0 ml of bi-distilled water containing 5% by weight of glycidyl-diisopropylidenxylitol-betacyclodextrin (GDX-bCD). A dispersion of 13 mg of digalactosyldiacylglycerol (DGDG) in 2.0 ml of bidistilled water was added under magnetic stirring to the dispersion of the microspheres. When the addition was finished, the dispersion was kept under magnetic stirring for a further period of 60 minutes. Dimensional analysis by means of dynamic light scattering showed that the microspheres were characterized by an average diameter of 200 nm and by a polydispersion index of 0.1 - 0.2.

### Example8

### Preparation of microspheres of PAM14/HSA/BSA-GAL-FITC

A solution of 200 mg of the butyl hemiester of the alternate copolymer of maleic anhydride/methoxyethyl vinyl ether (PAM14) in 4.0 ml of a 3/2 mixture of ethanol/water was dropped by means of a syringe fitted with a 22G needle into a solution of 40 mg of human seroalbumin (HSA) and 418 mg of bovine albumin galactopyranopkenylfluoresceinisothiocyanate (BSA-GAL-FITC) in 10 ml of bidistilled water. The dispersion of microspheres was kept under magnetic stirring for 50 minutes. Dimensional analysis by means of dynamic light scattering showed that the microspheres were characterised by an average diameter of 200 nm and by a poly-dispersion index of 0.1 - 0.2.

### Example 9

### Preparation of microspheres of PAM14/HSA/HSA-FITC/ASFET

A solution of 100 mg of the butyl hemiester of the alternate copolymer of maleic anhydride/methoxyethyl vinyl ether (PAM14) in 2.0 ml of a 3/2 mixture of ethanol/water was dropped by means of a syringe fitted with a 22G needle into a solution of 31 mg of human seroalbumin (HSA), 243 mg of human seroalbumin fluoresceinisothiocyanate (HSA-FITC) and 11 mg of asialofetuin (ASFET) in 5.0 ml of bidistilled water. The dispersion of microspheres was kept under magnetic stirring for 60 minutes. Dimensional analysis by means of dynamic light scattering showed that the microspheres were characterized by an average diameter of 1500 nm and by a polydispersion index of 0.6.

### Example 10

### Preparation of microspheres of PAM11/HSA/HGL/DGDG/HPCD

A solution of 250 mg of the methyl hemiester of the alternate copolymer of maleic anhydride/methoxyethyl vinyl ether (PAM11) in 10.0ml of a 1/20 mixture of ethanol/water was dropped by means of a syringe fitted with a 23G needle into a solution of 40 mg of human seroalbumin (HSA) in 25 ml of bidistilled water containing 5% by weight of hydroxypropylbetacyclodextrin (HPCD). A solution of 16 mg of 1-(3-dimethylaminopropyl)-3-ethylcarbodiimide hydrochloride in 2 ml of water was added to the dispersion of microspheres while keeping the dispersion under magnetic stirring for 2 minutes. Next a dispersion of 25 mg of a 1/1 mixture of galacto-lipid/hydrogenated galactolipid (DGDGHGL) in 5 ml of water heated up to 70°C was added to the suspension and magnetic stirring was kept on for 40 minutes. Dimensional analysis by means of dynamic light scattering showed that the microspheres were characterized by an average diameter of 600 nm.

### Example 11

### Measurements of delivery kinetics of proteins containing fluorescent markers from suspensions of microspheres

A dispersion of microspheres (5 ml) which are based on the butyl hemiester of the alternate copolymer of maleic anhydride/methoxyethyl vinyl ether (PAM14), human seroalbumin/human seroalbumin fluoresceinisothiocyanate (HSA/HSA-FITC) and digalactosyldiacylglycerol (DGDG), was put into a Spectra/Por dispodyalizer (diameter 10 mm, volume 5 ml, cut-off 100,000). The dispodyalizer was kept under magnetic stirring for 48 hours in 28 ml of a buffer solution of isotonic phosphate at pH 7.4, which had been put into a double-wall glass cylinder kept at 37°C. At determined time intervals 5 ml of the dyalizing solution were drawn and substituted with the same volume of fresh solution. The content of the dyalized protein was evaluated by fluorescence intensity measurements at 520 nm. Under the conditions employed, an amount of 405 of the total protein present in the dispersion of microspheres was delivered in 3 hours, with a typical "burst" effect, whereas a further amount of 505 was delivered at an almost constant rate within the next 40 hours.

### Example 12

### Measurements of delivery kinetics of proteins containing fluorescent markers from suspensions of microspheres

A dispersion of microspheres (5 ml) which were based on the butyl hemiester of the alternate copolymer of maleic anhydride/methoxyethyl vinyl ether (PAM14), human seroalbumin/bovine albumin galactopyranophenyl-fluoresceinisothiocyanate (HSA/BSA-GAL-FITC) and digalactosyldiacylglycerol (DGDG) was put into a Spectra/Por dispodyalizer (diameter 10 mm, volume 5 ml, cut-off 100,000). The dispodyalizer was kept under magnetic stirring for 48 hours in 28 ml of a buffer isotonic solution of phosphate at pH 7.4, in a double-wall glass cylinder kept at 37°C. At determined time intervals 5 ml of the dyalizing solution were drawn and substituted with the same volume of fresh solution. The content of the dyalized protein was evaluated by means of fluorescence intensity measurements at 520 nm. Under the conditions employed, an amount of 10% of the total protein present in the dispersion of the microspheres was delivered within the first hour, with a typical "burst" effect, whereas a further amount of 80% was delivered at an almost constant rate within the next 30 hours.

### Example 13

### Determination of the presence of superficial galactosyl residues by means of measurements of microparticle link with galactose-specific agglutinin lectin from Ricinus communis (RCA)

Lectin from *Ricinus communis* (Castor bean) agglutinin RCA (SIGMA) was employed, which had the following agglutination activity: 0.3 µg/ml of RCA120 were able to agglutinate a 26% v/v suspension of erythrocytes.

A suspension of microparticles based on the butyl hemiester of the alternate copolymer of maleic anhydride/methoxyethyl vinyl ether (PAM14), human seroalbumin (HSA) and digalactosyldiacylglycerol (DGDG) was centrifuged at 15,000 G for 15 minutes and the residue was re-suspended in an isotonic buffer solution of phosphate (PBXs) at pH 7.4. 1 ml aliquot parts of this suspension were transferred into a thermostatic cell and kept at 37°C for 15 minutes, under stirring. Transmittance of the microparticles samples was adjusted to 90% and 1 ml of a solution of RCA in PBS 6.66 mM at pH 7.4 was added. The change in optical density (O.D.) at 650 nm of the suspension was recorded and expressed as the percentage change with respect to the initial value. The results showed that an interaction of microparticles with RCA occurred, as is reported in table 1.

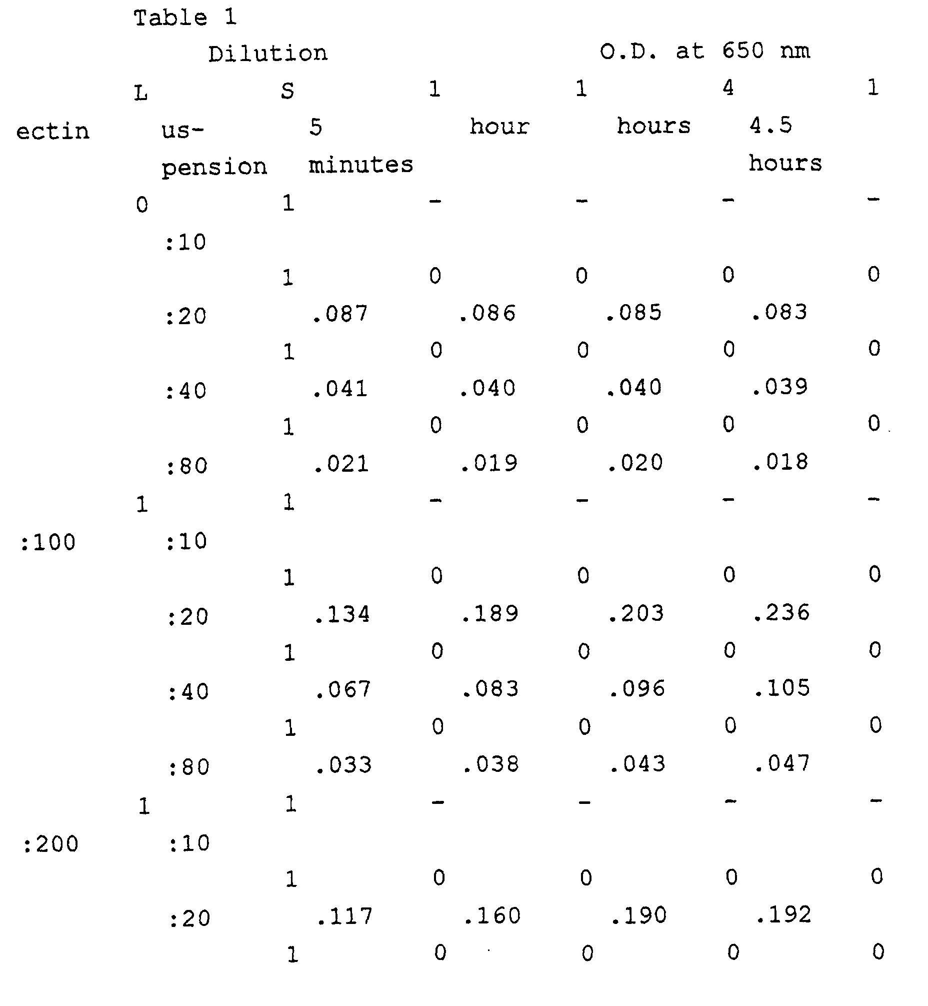

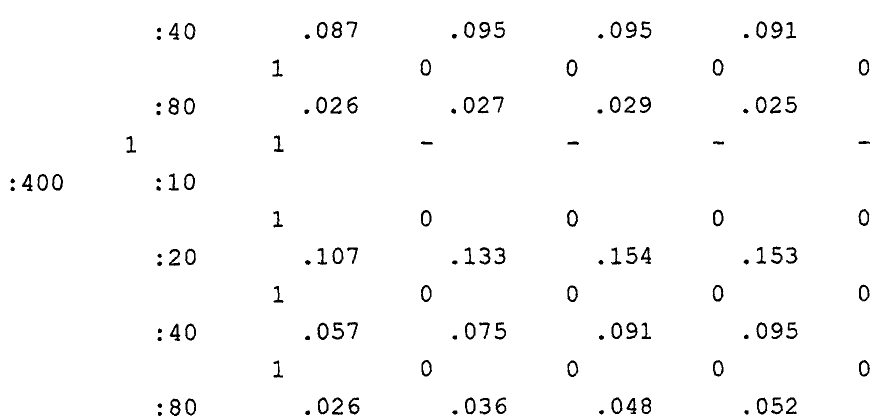

### Example 14

### Determination of the presence of superficial galactosyl residues by means of measurements of inhibition by microparticles of the agglutination of red blood cells (RBC) induced by agglutinin from Ricinus communis (RCA)

The assay is based on the competition between the galactose residues exposed both on red blood cells and on the microspheres for galactose-specific receptors which are present on the agglutinin from *Ricinus communis*.

Red blood cells from donors (group O, negative Rh) in dextrose citrate (ACD) were separated from blood by centrifugation at 3,000 rpm, then washed twice with physiological phosphate buffer (PBS) at pH 7.4 and finally re-suspended at a concentration of 1% v/v in PBS. Agglutinin from *Ricinus communis* (RCA) was diluted with PBS to obtain a standard solution containing 20 µg/ml of RCA. The agglutination titre of such reference standard solution was determined on a 96-well plate by adding 50µl of PBS, 50µl of the control solution and 100µl of red blood cell suspension. After two hours at room temperature, the agglutination titre and the "agglutination unit" (i.e. the highest dilution capable of agglutination effect) were evaluated visually.

Next, 50µl of PBS, 50µl of serial dilutions with doubling of microparticle suspension, 50µl of RCA control solution (1 and 4 agglutination units) were put in this order into the wells of a 96-well plate, and after 30 minutes of incubation at room temperature 100µl of erythrocyte suspension were added. After one hour of incubation at room temperature the plates were inspected visually and the highest dilution of the suspension of microparticles that was able to inhibit agglutination of red blood cells was evaluated as 1/32 when 1 unit of agglutination was employed and as 1/16 when 4 units of agglutination were employed.

A confirmation assay was carried out employing D(+) galactose as an inhibitor of RCA-induced agglutination of red blood cells.

### Example 15

### Interaction of microparticles with hepatocyte receptors for asialoglycoproteins

The interaction of the galactosyl residues present on the surface of the PAM14HSA/HSA-FITC/DGDG/GDA-bCD microparticles prepared in example 6 with the hepatic cell receptors specific for galactose was evaluated by means of measurements of flow cytofluorometry employing rat hepatocytes.

The microparticles were isolated by centrifugation of the suspension at 14,000 G, then extensively washed with saline solution and finally re-suspended in PBS at about 240 mg/ml. Rat hepatic cells were prepared by perfusion with collagenase according to the method by Seglen, Methods Cell. Biol. 18: 29 (1976), and grown at 37°C on Eagle soil, modified according to Dulbecco (DMEM) added with 0.1% albumin in a 5% carbon anhydride environment. Hepatocytes were incubated with 240 mg/ml of suspension of microparticles for 2 hours at 37°C in DMEM added with 0.9 mM Ca⁺² in a 5% carbon anhydride environment. Employing a FACS analyzer (Becton and Dickinson) the fluorescence of each cell was recorded at a rate of 300 cells/sec (excitation wavelength 488 nm by means of an argon ion laser and emission wavelength 515 nm). Dead cells were identified and counted after incubation with 5µg/ml of propidium chloride (negative control). A statistical processing of data according to Kolmogorov-Smirnov, J. Histochem Cytochem. 25: 935 (1977) gave a value of 0.21 for the coefficient D, and D/s(n) = 11.06, with a positive value of threshold: D/s(n) = 8.

### Example 16

### Interaction of microspheres with human immunoglobulins or human seroalbumin

Lack of capability of the microspheres to interact with opsonizing proteins like the immunoglobulins G (IgG) and human seroalbumin (HSA) was evaluated by determining the proteins not bonded after incubation at 37°C for different periods of time. Polystyrene microparticles, diameter 305 nm, were employed as probes and as a reference standard.

The samples of suspensions of microspheres whose surface area was 0.05 square metres/ml, diluted with physiological solution, were incubated in Rppendorf couvettes with IgG or HSA solutions. After 2 or 3 hours of incubation at 37°C and separation by means of an Eppendorf centrifuge at 15,000 G for 2 hours, the supernatants in the samples were drawn and their protein content was measured by means of spectrophotometry (Tables 2a and 2b).

HSA: max: 277 nm; absorbance of a 1% solution = 5.7 (1 = 1 cm)

IgG: max 279nm; absorbance of a 1% solution = 13.8 (1 = 1 cm)

**Table 2a**

| Absorption of HSA on microspheres | | | |
|---|---|---|---|
| Free HSA | | Absorbed HSA | |
| µg/ml | µg | mg/m² | % (1) |
| 962 | 31.6 | 0.632 | 15.8 |
| | 32.1 | 0.656 | 16.4 |
| 466 | 32 | 0.642 | 16.05 |
| | 33 | 0.642 | 16.05 |
| 217 | 31.8 | 0.636 | 15.9 |
| | 34 | 0.680 | 17 |

**Table 2b**

| Absorption of IgGs on microspheres | | | |
|---|---|---|---|
| Free IgGs | | IgGs Absorbed | |
| µg/ml | µg | mg/m² | % (1) |
| 1250 | 33.2 | 0.664 | 18.9 |
| | 34.8 | 0.696 | 19.8 |
| 614 | 34.4 | 0.688 | 19.6 |
| | 35.7 | 0.895 | 25.6 |
| 290 | 34.7 | 0.696 | 19.9 |
| | 38.2 | 0.955 | 27.3 |

| | | | |
|---|---|---|---|
| (1) Percentage of protein adsorbed on the sample with respect to polystyrene. | | | |

### Example 17

### A quantitative evaluation of the content of galactose residues

The galactose residue content in the various microspheres samples was evaluated by colorimetric determination of the reaction products of galactose with phenol in sulphuric acid [(Dubiois et al., Colorimetric method for determination of sugars and related substances, Anal. Chem., 28: 350-356 (1956)].

The samples to analyze were diluted to a concentration of galactose residues from 10 to 50 µg/ml. 2 ml of the solution to assay, 1 ml of a solution containing 5.0 g of phenol in 100 ml of water and 5ml of 96% sulphuric acid were put into a 20 ml test tube in the order specified herein. After 10 minutes the resulting solution was mixed strongly and then heated up to 35-40°C for 10 minutes. The galactose content was evaluated by comparing the solution absorbance at 490 nm with the absorbance of the reaction products of standard solutions containing about 10, 30, 50, 70 and 90 µg/ml of galactose.

For comparison, the determination of galactose was also performed on the individual components employed for the preparation of microspheres, i.e. distilled water, PAM14, DGDG,betaCD-GDA, HSA, HSA-FITC and BSA-GAL-FITC.

The results obtained show that 5% of the galactose residues and so of the DGDG glycolipids is present in the centrifuged pellet.

### Example 18

Microencapsulation of leucocyte interferon into the microspheres

A solution of 210 mg of the butyl hemiester of the alternate copolymer of maleic anhydride/methoxyethyl vinyl ether (PAM14) in 4 ml of a 4/1 mixture of ethanol/water was dropped by means of a syringe fitted witha 22G needle into a solution of 110 mg of human albumin (HSA) and 3,000,000 I.U. of human alpha-leucocyte interferon (IFNa) in 10 ml of bidistilled water containing 5% by weight of glycidyldiisopropylidene-arabitol-betacyclodextrin.

When the addition was finished, the dispersion was kept under magnetic stirring for 60 minutes. A dispersion of 25 mg of a 1/1 mixture of galactolipid/hydrogenated galactolipid was added during a period of 10 minutes to the suspension so obtained, kept under magnetic stirring. Stirring was kept on for 40 minutes.

The determination of microsphere inhibition of the cytopatic effect caused by the bovine stomatitis virus on Wish cells, by working according to E.A. Havell et al., Antimicrob. Agents Chemother. 2: 476 (1972), allowed a value of incorporation efficiency of IFNa equal to 90% to be determined.

## Claims

1. A chemically homogeneous microparticle for controlled delivery of biologically active molecules, said microparticle being made up of:
i) a core comprising a proteic component and a natural, synthetic or semisynthetic polymer, said proteic component and said polymer having non-covalent and/or ionic-type interactions and forming an homogeneous net, and
ii) an outside layer consisting of natural, synthetic or semisynthetic molecules that are capable of being recognized by receptors or components of the cell surface of living organisms, or of recognizing natural, synthetic or semisynthetic molecular structures;
the microparticle having a size between 100 nm and 1,000 nm, preferably between 100 nm and 400 nm, with a monomodal distribution and limited-value polydispersion index.

2. A chemically homogeneous microparticle according to claim 1, wherein the essentially proteic component is made up of at least a protein selected from among albumin (human, bovine or egg albumin), alpha-1 globulin, alpha-2 globulin, collagen, fibrinogen, gelatin.

3. A chemically homogeneous microparticle according to claim 2, wherein said protein is natural or recombinant human albumin.

4. A chemically homogeneous microparticle according to anyone of the preceding claims, wherein the core further comprises modified cyclodextrins.

5. A chemically homogeneous microparticle according to any one of the preceding claims, wherein the natural, synthetic or semisynthetic polymer is made up of polyanionic polymers and their mixtures.

6. A chemically homogeneous microparticle according to claim 5, wherein said polyanionic polymers contain carboxyl and/or sulfonic and/or phosphoric groups.

7. A chemically homogeneous microparticle according to any one of the preceding claims, wherein said polymer is made up of hemiesters of alternate copolymers of maleic anhydride with alkylvinyl ethers oxyethylene-glycol with polymerization degree 1≤n≤1000.

8. A chemically homogeneous microparticle according to claim 7, wherein the polymerization degree is 1≤n≤10.

9. A chemically homogeneous microparticle according to any one of the preceding claims, wherein said outside layer is made up of glycolipids and/or glyco-proteins and/or synthetic or semisynthetic polymers which contain glycosidic or saccharidic residues, and/or mono- and/or oligosaccharides.

10. A chemically homogeneous microparticle according to claim 9, wherein said glycosidic residues are galactose, lactose or mannose residues.

11. A chemically homogeneous microparticle according to claim 9, wherein the outside layer is made up of polyamino acids containing galactose, lactose or mannose residues.

12. A chemically homogeneous microparticle according to claim 9, wherein the glycolipids are made up of mono- or digalactosyldiglycerides, gangliosides, lactosylverebrosides, neogalactolipids (lipids modified by means of saccharide residues).

13. A chemically homogeneous microparticle according to claim 9 , wherein said glycoproteins are made up of asialoglycoproteins and neoglycoproteins.

14. A chemically homogeneous microparticle according to claim 13, wherein the asialoglycoproteins comprise asialofetuin, asialoorosomucoid, asialotransferrin.

15. A chemically homogeneous microparticle according to claim 9, wherein said glycoproteins comprise glycosylated albumin, and in particular galactosylated, lactosylated and mannosylated albumin.

16. A chemically homogeneous microparticle according to claim 9, wherein said mono an/or oligosaccharides are made up of galactose, lactose and/or mannose.

17. A chemically homogeneous microparticle according to any one of the preceding claims, wherein said receptors or components of the cell surface of living organisms are expressed under normal or pathological conditions on hepatic, parenchymal and non-parenchymal, cells or in the bone marrow.

18. A chemically homogeneous microparticle according to claim 17, wherein said receptors are of the selectinic E or P type.

19. A chemically homogeneous microparticle according to any one of the preceding claims, wherein said biologically active molecules are made up of peptides, polypeptides, proteins, carbohydrates, nucleic acids, lipids or polysaccharides, be the natural, synthetic or semisynthetic.

20. A chemically homogeneous microparticle according to claim 19, wherein said nucleic acids comprise polynucleotides, antisense oligonucleotides, molecular conjugates containing RNA or DNA, water soluble RNA or DNA vectors.

21. A chemically homogeneous microparticle according to claim 19, wherein said polypeptides comprise cytochins, lymphochins, monochins, interferons.

22. A chemically homogeneous microparticle according to claim 21, wherein said interferon is natural, synthetic or recombinant alpha-interferon.

23. A chemically homogeneous microparticle according to any one of the preceding claims 1 - 18, wherein said biologically active molecules are a natural or a recombinant vaccine.

24. A chemically homogeneous microparticle according to any one of the preceding claims 1 - 18, wherein said biologically active molecules are made up of bacterial or viral antigens, natural or recombinant.

25. A chemically homogeneous microparticle according to any one of the preceding claims 1 - 18, wherein said biologically active molecules are an immunological adjuvant.

26. A chemically homogeneous microparticle according to any one of the preceding claims 1 - 18, wherein said biologically active molecules are made up of or are linked to natural or recombinant antibodies and/or antibody fragments.

27. A chemically homogeneous microparticle according to any one of the preceding claims 1 - 18, wherein said biologically active molecules are made up of a radioactive agent, an enzyme, a coagulation factor, or a water soluble hormone.

28. A chemically homogeneous microparticle according to claim 27, wherein said enzyme is the superoxide-dismutase, even modified.

29. A chemically homogeneous microparticle according to claim 27, wherein said coagulation factor is the VIII factor.

30. A chemically homogeneous microparticle according to any one of the preceding claims 1 - 18, wherein said biologically active molecules are made up of natural, synthetic or semisynthetic substances belonging to antitumorals, antibiotics, antiinflammatories, immuno-stimulants, immuno-suppressants.

31. A chemically homogeneous microparticle according to claim 1, wherein the targeting towards specific organs is obtained on the basis of the average size of microparticles.

32. A process for preparing microparticles according to any one of the preceding claims, by means of:
- mixing a water hydroalcoholic organic solution of a natural, synthetic or semisynthetic polymer with a proteic solution;
- performing a controlled co-precipitation at a pH between 3 and 7, preferably between 4 and 5, at a temperature variable between 0°C and 70°C, preferably between 10 and 40°C;
- adding the component of the outside layer under stirring;
- stirring until evaporation of the organic solvent.

33. A pharmaceutical composition comprising microparticles according to any one of the claims 1 - 31, which are dispersed in a pharmaceutically acceptable medium.

34. A pharmaceutical composition according to claim 33, in the form of a lyophilized substance for reconstitution, of a water suspension or of a gel.

35. A pharmaceutical composition according to claim 33, for administration by injection and/or implantation by the subcutaneous, intradermal, intrathecal,intramuscular,intraperitoneal, intravenous, intra-arterial routes.

## Patentansprüche

1. Chemisch homogener Mikropartikel zur gesteuerten Wirkstofffreigabe biologisch aktiver Moleküle, bestehend aus:
i) einem Kern bestehend aus einem Proteinbestandteil und einem natürlichen, synthetischen oder halbsynthetischen Polymer, wobei genannter Proteinbestandteil und genanntes Polymer nichtkovalente und/oder ionische Wechselwirkungen aufweisen und ein homogenes Netz bilden, und
ii) einer Außenschicht bestehend aus natürlichen, synthetischen oder halbsynthetischen Molekülen, die durch Rezeptoren oder Verbindungen auf der Zelloberfläche von lebenden Organismen erkannt werden können, oder aus natürlichen, synthetischen oder halbsynthetischen molekularen Erkennungsstrukturen;
wobei die Mikropartikel eine Größe zwischen 100 nm und 1.000 nm, vorzugsweise zwischen 100 nm und 400 nm mit einer monomodalen Verteilung und einem Grenzwert-Polydiserpersionsindex aufweisen.

2. Chemisch homogener Mikropartikel nach Anspruch 1, wobei der wesentliche Proteinbestandteil aus mindestens einem Protein ausgewählt aus Albumin (human, bovin oder Eialbumin), α1-Globulin, α2-Globulin, Collagen, Fibrinogen oder Gelatine besteht.

3. Chemisch homogener Mikropartikel nach Anspruch 2, wobei genanntes Protein ein natürliches oder rekombinantes Humanalbumin ist.

4. Chemisch homogener Mikropartikel nach einem der vorhergehenden Ansprüche, wobei der Kern weiterhin modifizierte Cyclodextrine umfasst.

5. Chemisch homogener Mikropartikel nach einem der vorhergehenden Ansprüche, wobei das natürliche, synthetische oder halbsynthetische Polymer aus polyanionischen Polymeren und deren Mischungen hergestellt wird.

6. Chemisch homogener Mikropartikel nach Anspruch 5, wobei das genannte polyanionische Polymer Carboxyl- und/oder Sulfonsäure- und/oder Phosphorsäuregruppen enthält.

7. Chemisch homogener Mikropartikel nach einem der vorhergehenden Ansprüche, wobei genanntes Polymer aus Halbestern von alternierenden Copolymeren aus Maleinsäureanhydrid und Oxyethylenglykol-alkylvinylethern mit einem Polymerisationsgrad von 1 ≤ n ≤ 1000 hergestellt wird.

8. Chemisch homogener Mikropartikel nach Anspruch 7, wobei der Polymerisationsgrad 1 ≤ n ≤ 10 beträgt.

9. Chemisch homogener Mikropartikel nach einem der vorhergehenden Ansprüche, wobei genannte äußere Schicht aus Glykolipiden und/oder Glykoproteinen und/oder synthetischen oder halbsynthetischen Polymeren hergestellt wird, die glykosidische oder saccharidische Reste und/oder Mono- und/oder Oligosaccharide enthalten.

10. Chemisch homogener Mikropartikel nach Anspruch 9, wobei genannte glykosidische Reste Galactose-, Lactose- oder Mannosereste sind.

11. Chemisch homogener Mikropartikel nach Anspruch 9, wobei die äußere Schicht aus Polyaminosäuren hergestellt wird, die Galactose-, Lactose- oder Mannosereste enthalten.

12. Chemisch homogener Mikropartikel nach Anspruch 9, wobei die Glykolipide bestehen aus Mono- oder Digalactosyldiglyceriden, Gangliosiden, Lactosylcerebrosiden, Neogalactolipiden (wobei die Lipide mittels Saccharidresten modifiziert werden).

13. Chemisch homogener Mikropartikel nach Anspruch 9, wobei genannte Glykoproteine bestehen aus Asialoglykoproteinen und Neoglykoproteinen.

14. Chemisch homogener Mikropartikel nach Anspruch 13, wobei die Asialoglykoproteine Asialofetuin, Asialoorosomukoid und Asialotransferrin umfassen.

15. Chemisch homogener Mikropartikel nach Anspruch 9, wobei genannte Glykoproteine glykosyliertes Albumin und insbesondere galactosyliertes, lactosyliertes und mannosyliertes Albumin umfassen.

16. Chemisch homogener Mikropartikel nach Anspruch 9, wobei genannte Monound/oder Oligosaccharide bestehen aus Galactose, Lactose und/oder Mannose.

17. Chemisch homogener Mikropartikel nach einem der vorhergehenden Ansprüche, wobei genannte Rezeptoren oder Komponenten der Zelloberfläche von lebenden Organismen unter normalen oder pathologischen Bedingungen auf hepatischen, parenchymalen und nicht-parenchymalen Zellen oder im Knochenmark expressiert werden.

18. Chemisch homogener Mikropartikel nach Anspruch 17, wobei genannte Rezeptoren vom Typ E- oder P-Selektin sind.

19. Chemisch homogener Mikropartikel nach einem der vorhergehenden Ansprüche, wobei genannte biologisch aktive Moleküle bestehen aus Peptiden, Polypeptiden, Proteinen, Kohlenhydraten, Nukleinsäuren, Lipiden oder Polysacchariden, und zwar in natürlicher, synthetischer oder halbsynthetischer Form.

20. Chemisch homogener Mikropartikel nach Anspruch 19, wobei genannte Nukleinsäuren Polynukleotide, Antisense-Oligonukleotide, RNA oder DNA enthaltende molekulare Konjugate oder wasserlösliche RNA- oder DNA-Yektoren umfassen.

21. Chemisch homogener Mikropartikel nach Anspruch 19, wobei genannte Polypeptide Cytokine, Lymphokine, Monokine oder Interferone umfassen.

22. Chemisch homogener Mikropartikel nach Anspruch 21, wobei genanntes Interferon ein natürliches, synthetisches oder rekombinantes α-Interferon ist.

23. Chemisch homogener Mikropartikel nach einem der vorhergehenden Ansprüche 1 - 18, wobei genannte biologische aktive Moleküle natürliche oder rekombinante Vakzine sind.

24. Chemisch homogener Mikropartikel nach einem der vorhergehenden Ansprüche 1 - 18, wobei genannte biologisch aktive Moleküle aus bakteriellen oder viralen, natürlichen oder rekombinanten Antigenen hergestellt werden.

25. Chemisch homogener Mikropartikel nach einem der vorhergehenden Ansprüche 1 - 18, wobei genannte biologisch aktive Moleküle ein immunologisches Adjuvans ist.

26. Chemisch homogener Mikropartikel nach einem der vorhergehenden Ansprüche 1 - 18, wobei genannte biologisch aktive Moleküle hergestellt werden aus oder verknüpft werden mit natürlichen oder rekombinanten Antikörpern und/oder Antikörperfragmenten.

27. Chemisch homogener Mikropartikel nach einem der vorhergehenden Ansprüche 1 - 18, wobei genannte biologisch aktive Moleküle hergestellt werden aus einem radioaktiven Wirkstoff, einem Enzym, einem Koagulationsfaktor oder einem wasserlöslichen Hormon.

28. Chemisch homogener Mikropartikel nach Anspruch 27, wobei genanntes Enzym, gegebenenfalls auch modifizierte, Superoxiddismutase ist.

29. Chemisch homogener Mikropartikel nach Anspruch 27, wobei genannter Koagulationsfaktor Faktor VIII ist.

30. Chemisch homogener Mikropartikel nach einem der vorhergehenden Ansprüche 1 - 18, wobei genannte biologisch aktive Moleküle hergestellt werden aus natürlichen, synthetischen oder halbsynthetischen Substanzen, die zu den Zytostatika, Antibiotika, entzündungshemmenden Wirkstoffen, Immunstimulantien oder Immunsuppressiva gehören.

31. Chemisch homogener Mikropartikel nach Anspruch 1; wobei die Zielorientierung auf bestimmte Organe auf Basis der durchschnittlichen Größe der Mikropartikel erreicht wird.

32. Verfahren zur Herstellung von Mikropartikeln nach einem der vorhergehenden Ansprüche mittels:
- Mischen einer wässrigen hydroalkoholischen organischen Lösung eines natürlichen, synthetischen oder halbsynthetischen Polymers mit einer proteinhaltigen Lösung;
- Ausführung einer kontrollierten Mitfällung bei einem pH-Wert zwischen 3 und 7, vorzugsweise zwischen 4 und 5; bei einer variablen Temperatur zwischen 0 °C und 70 °C, vorzugsweise zwischen 10 °C und 40 °C;
- Zugabe des Bestandteils für die äußere Schicht unter Rühren;
- Rühren bis zum Verdampfen des organischen Lösungsmittels.

33. Pharmazeutische Zusammensetzung umfassend Mikropartikel nach einem der Ansprüche 1 - 31, welche in einem pharmazeutische zulässigem Medium dispergiert wird.

34. Pharmazeutische Zusammensetzung nach Anspruch 33 in Form einer lyophilisierten Substanz zur Rekonstitution in einer wässrigen Suspension oder einem Gel.

35. Pharmazeutische Zusammensetzung nach Anspruch 33, zur Verabreichung mittels Injektion und/oder Implantation über subkutane, intrakutane, intrathekale, intramuskuläre, intraperitoneale, intravenöse oder intraarterielle Wege.

## Revendications

1. Microparticule chimiquement homogène pour une délivrance contrôlée de molécules biologiquement actives, ladite microparticule étant formée de:
i) un coeur comprenant un composant protéique et un polymère naturel, synthétique ou semi-synthétique, ledit composant protéique et ledit polymère ayant des interactions du type non-covalent et/ou ionique et formant un réseau homogène, et
ii) une couche extérieure consistant en molécules naturelles, synthétiques ou semi-synthétiques qui sont capables d'être reconnues par des récepteurs ou composants de la surface de cellule d'organismes vivants, ou de reconnaître des structures moléculaires naturelles, synthétiques ou semi-synthétiques;
la microparticule ayant une dimension comprise entre 100 nm et 1000 nm, de préférence entre 100 nm et 400 nm, avec une distribution monomodale et un indice de polydispersion de valeur limitée.

2. Microparticule chimiquement homogène selon la revendication 1, où le composant essentiellement protéique est formé d'au moins une protéine sélectionnée parmi albumine (albumine humaine, bovine ou de l'oeuf), alpha-1 globuline, alpha-2 globuline, collagène-fibrinogène, gélatine.

3. Microparticule chimiquement homogène selon la revendication 2, où ladite protéine est l'albumine humaine naturelle ou recombinante.

4. Microparticule chimiquement homogène selon l'une quelconque des revendications précédentes, où le coeur comprend de plus des cyclodextrines modifiées.

5. Microparticule chimiquement homogène selon l'une quelconque des revendications précédentes, où le polymère naturel, synthétique ou semi-synthétique est formé de polymères polyanioniques et leurs mélanges.

6. Microparticule chimiquement homogène selon la revendication 5, où lesdits polymères polyanioniques contiennent des groupes carboxyles et/ou sulfoniques et/ou phosphoriques.

7. Microparticule chimiquement homogène selon l'une quelconque des revendications précédentes, où ledit polymère est formé d'hémi-esters de copolymères alternés d'anhydride maléique avec des alkylvinyl éthers oxyéthyiène-glycol avec un degré de polymérisation de 1≤n≤1000.

8. Microparticule chimiquement homogène selon la revendication 7, où le degré de polymérisation est 1≤n≤10.

9. Microparticule chimiquement homogène selon l'une quelconque des revendications précédentes, où ladite couche extérieure est formée de glycolipides et/ou glycoprotéines et/ou polymères synthétiques ou semi-synthétiques qui contiennent des résidus glycosidiques ou saccharidiques, et/ou des mono- et/ou oligosaccharides. 9,

10. Microparticule chimiquement homogène selon la revendication 9, où lesdits résidus glycosidiques sont des résidus de galactose, lactose ou mannose.

11. Microparticule chimiquement homogène selon la revendication 9, où la couche extérieure est formée d'acides polyaminés contenant des résidus de galactose, lactose ou mannose.

12. Microparticule chimiquement, homogène selon la revendication 9, où les glycolipides sont formés de mono- ou digalactosyldiglycéridés, gangliosides, lactosylvéré-brosides, néogalactolipides (lipides modifiés, au moyen de résidus de saccharides).

13. Microparticule chimiquement homogène selon la revendication 9, où lesdites glycoprotéines sont formées d'asialoglycoprotéines et de néoglycoprotéines.

14. Microparticule chimiquement homogène selon la revendication 13, où les asialoglycoprotéines comprennent asialofétuine, asialoorosomucoïde, asialotransferrine.

15. Microparticule chimiquement homogène selon la revendication 9, où lesdites glycoprotéines comprennent de l'albumine glycosylée, et en particulier, de l'albumine galactosylée, lactosylée et mannosylée.

16. Microparticule chimiquement homogène selon la revendication 9, où lesdits mono- et/ou oligosaccharides sont formés de galactose, lactose et/ou mannose.

17. Microparticule chimiquement homogène selon l'une quelconque des revendications précédentes, où lesdits récepteurs ou composants de la surface de cellule d'organismes vivants sont exprimés en conditions normales ou pathologiques sur des cellules hépatiques, parenchymales et non-parenchymales ou dans la moëlle osseuse.

18. Microparticule chimiquement homogène selon la revendication 17, où lesdits récepteurs sont du type sélectinique E ou P.

19. Microparticule chimiquement homogène selon l'une quelconque des revendications précédentes, où lesdites molécules biologiquement actives sont formées de peptides, polypeptides, protéines, carbohydrates, acides nucléiques, lipides ou polysaccharides, qu'ils soient naturels, synthétiques ou semi-synthétiques.

20. Microparticule chimiquement homogène selon la revendication 19, où lesdits acides nucléiques comprennent des polynucléotides, des oligonucléotides anti-sens, des conjugués moléculaires contenant de l'ARN ou de l'ADN, des vecteurs d'ADN ou d'ARN solubles dans l'eau.

21. Microparticule chimiquement homogène selon la revendication 19, où lesdits polypeptides comprennent des cytochines, des lymphochines, des monochines, des interférons.

22. Microparticule chimiquement homogène selon la revendication 21, où ledit interféron est l'alpha-interféron naturel, synthétique ou recombinant.

23. Microparticule chimiquement homogène selon l'une quelconque des revendications précédentes 1-18, où lesdites molécules biologiquement actives sont un vaccin naturel ou recombinant.

24. Microparticule chimiquement homogène selon l'une quelconque des revendications précédentes 1-18, où lesdites molécules biologiquement actives sont formées d'antigènes bactériens ou viraux, natures ou recombinants.

25. Microparticule chimiquement homogène selon l'une quelconque des revendications précédentes 1-18, où lesdites molécules biologiquement actives sont un adjuvant immunologique.

26. Microparticule chimiquement homogène selon l'une quelconque des revendications précédentes 1-18, où lesdites molécules biologiquement actives sont formées de ou sont enchaînées à des anticorps naturels ou recombinants et/ou fragments d'anticorps.

27. Microparticule chimiquement homogène selon l'une quelconque des revendications précédentes 1-18, où lesdites molécules biologiquement actives sont formées d'un agent radioactif, d'une enzyme, d'un facteur de coagulation ou d'une hormone soluble dans l'eau.

28. Microparticule chimiquement homogène selon la revendication 17, où ladite enzyme est la superoxyde-dismutase, même modifiée.

29. Microparticule chimiquement homogène selon la revendication 27, où ledit facteur de coagulation est le facteur VIII.

30. Microparticule chimiquement homogène selon l'une quelconque des revendications précédentes 1-18, où lesdites molécules biologiquement actives sont formées de substances naturelles, synthétiques ou semi-synthétiques appartenant aux antitumoraux, antibiotiques, anti-inflammatoires, immuno-stimulants, immuno-suppresseurs.

31. Microparticule chimiquement homogène selon la revendication 1, où le ciblage vis-à-vis d'organes spécifiques est obtenu sur la base de la dimension moyenne des microparticules.

32. Procédé de préparation de microparticules selon l'une quelconque des revendications précédentes, au moyen de:
- mélange d'une solution organique hydroalcoolisée dans l'eau, d'un polymère naturel, synthétique ou semi-synthétique avec une solution protéique;
- accomplissement d'une co-précipitation contrôlée à un pH entre 3 et 7, de préférence entre 4 et 5, à une température variable entre 0°C et 70°C, de préférence entre 10°C et 40°C;
- addition du composant de la couche extérieure sous agitation;
- agitation jusqu'à évaporation du solvant organique.

33. Composition pharmaceutique comprenant des microparticules selon l'une quelconque des revendications 1-31, qui sont dispersées dans un support pharmaceutiquement acceptable.

34. Composition pharmaceutique selon la revendication 33, sous la forme d'une substance lyophilisée pour reconstitution d'une suspension dans l'eau ou d'un gel.

35. Composition pharmaceutique selon la revendication 33, pour administration par injection et/ou implantation par les voies sous-cutanée, intradermique, intrathécale, intramusculaire, intrapéritonéale, intraveineuse, intra-artérielle.
